# EUROPEAN PATENT APPLICATION

(11) **EP 1 983 345 A1**
(43) Date of publication of application: **22.10.2008**
(21) Application number: 07106306.9
(22) Date of filing: 17.04.2007
(51) Int. Cl.: G01N 33/68

(54) **Means and method for assessing the risk of a cardiac intervention in patients suffering from a stable coronary heart disease based on GDF-15**

(71) Applicant: Roche Diagnostics GmbH, 68305 Mannheim (DE); F. Hoffmann-La Roche AG, 4070 Basel (CH)
(72) Inventor: Hess, Georg, Prof. Dr., 55130 Mainz (DE); Horsch, Andrea, Dr., 68259 Mannheim (DE); Zdunek, Dietmar, Dr., 82327 Tutzing (DE)
(74) Representative: Dick, Alexander

(57) **Abstract**

The present invention relates to a method for diagnosing whether a percutaneous cardiac intervention (PCI) in a subject suffering from a stable coronary heart disease was successful, said method comprising determining the amount of GDF-15 in a first sample of the said subject which has been obtained after PCI and comparing the determined amount of GDF-15 with a reference amount of GDF-15 which is determined in a second sample of the said subject suffering from a stable coronary heart disease obtained prior to PCI, whereby it is diagnosed whether PCI was successful. Further, the present invention relates to the use of means for determining the amount of GDF-15 and, preferably, a natriuretic peptide and/or a cardiac Troponin for the preparation of a diagnostic composition for diagnosing whether a percutaneous cardiac intervention (PCT) in a subject suffering from a stable coronary heart disease was successful. The present invention also encompasses a device and a kit adopted for diagnosing whether a percutaneous cardiac intervention (PCI) in a subject suffering from a stable coronary heart disease was successful.

## Description

The present invention concerns the field of diagnostic means and methods for risk stratification. Specifically, the present invention relates to a method for diagnosing whether a percutaneous cardiac intervention (PCI) in a subject suffering from a stable coronary heart disease was successful, said method comprising determining the amount of GDF-15 in a first sample of the said subject which has been obtained after PCI and comparing the determined amount of GDF-15 with a reference amount of GDF-15 which is determined in a second sample of the said subject suffering from a stable coronary heart disease obtained prior to PCI, whereby it is diagnosed whether PCI was successful. Further, the present invention relates to the use of means for determining the amount of GDF-15 and, preferably, a natriuretic peptide and/or a cardiac Troponin for the preparation of a diagnostic composition for diagnosing whether a percutaneous cardiac intervention (PCI) in a subject suffering from a stable coronary heart disease was successful. The present invention also encompasses a device and a kit adopted for diagnosing whether a percutaneous cardiac intervention (PCI) in a subject suffering from a stable coronary heart disease was successful.

Growth-differentiation factor-15 (GDF-15) has been suggested to be an indicator for cardiovascular complications (US2003/0232385; Kempf 2006, Circ Res 98: 351-360). GDF-15 is a member of the transforming growth factor-β cytokine superfamily. GDF-15 was first identified as macrophage-inhibitory cytokine-1 (MIC-1) and later also named placental transforming growth factor-β (Bootcov 1997, Proc Natl Acad Sci 94:11514-11519; Tan 2000, Proc Natl Acad Sci 97:109-114). It has recently been shown that cultured cardiomyocytes express and secrete GDF-15 via nitric oxide and nitrosative stress-dependent signaling pathways when subjected to simulated ischemia and reperfusion. Moreover, it has been observed in a mouse model of myocardial ischemia and reperfusion injury that GDF-15 expression levels rapidly increase in the ischemic area following coronary artery ligation, and remain elevated in the reperfused myocardium for several days (Kempf loc. cit.).

Patients suffering from atherosclerosis or other occlusive disorders of the coronary heart vessels usually require a reperfusion therapy such as a percutaneous cardiac intervention. These interventions include stent implantation therapies. For stent implantation, a stent device will be placed at the side of stenosis into the coronary heart vessel. The stent will be subsequently fixed to the vessel wall by balloon extension. The balloon extension, however, causes a temporary occlusion of the vessel. After the stent has been fixed, the balloon will be removed and reperfusion occurs provided the implantation was successful. In case, the implantation was unsuccessful, reperfusion is abolished due to embolism which may occur at the side of implantation or even a thrombo-embolic occlusion. These are life-threatening conditions which require immediate attention and supportive care.

At present, the success of a percutaneous cardiac intervention is determined by the reperfusion as detected by angiographic techniques. However, the angiographic techniques itself may cause adverse side effects for the patient. Moreover, these techniques can be, usually, only applied during or immediately after the implantation process and are, thus, not suitable for monitoring.

Thus, reliable and efficient means and methods for determining and monitoring the reperfusion efficacy and, thus, the success of the percutaneous cardiac intervention, are not yet available but nevertheless highly desirable. There is a need for diagnostic or prognostic measures which allow for reliable and fast risk stratification in a patient suffering from a stable coronary heart disease which has been subjected to a percutaneous cardiac intervention, such as stent implantation.

The technical problem underlying the present invention can be seen as the provision of means and methods for complying with the aforementioned needs. The technical problem is solved by the embodiments characterized in the claims and herein below.

Accordingly, the present invention relates to a method for diagnosing whether a percutaneous cardiac intervention (PCI) in a subject suffering from a stable coronary heart disease was successful, said method comprising:
a) determining the amount of GDF-15 in a first sample of the said subject which has been obtained after PCI; and
b) comparing the amount of GDF-15 determined in step a) with a reference amount of GDF-15 which is determined in a second sample of the said subject suffering from a stable coronary heart disease obtained prior to PCI, whereby it is diagnosed whether PCI was successful.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of the subject. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

The term "diagnosing" as used herein means assessing as to whether a percutaneous cardiac intervention was successful. As will be understood by those skilled in the art, such an assessment is usually not intended to be correct for all (i.e. 100%) of the subjects to be investigated. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly assessed by the method of the present invention.

Diagnosing according to the present invention includes monitoring, confirmation, subclassification in connection with the assessment. Monitoring relates to keeping track of the markers and, thus, allows reassessing the success at different time points after the intervention. Confirmation relates to the strengthening or substantiating a prior assessment including assessments made by using other indicators or markers. Subclassification relates to further specify the assessment according to different success subclasses.

The term "percutaneous cardiac intervention (PCI)" relates to those treatment regimens which comprise an intervention by surgery, microsurgery or other invasive therapies affecting the cardiovascular system and, preferably, the heart. Preferably, percutaneous cardiac interventions as used herein are treatment regimens which aim to restore the proper oxygen supply of the heart. This is, preferably, achieved by restoring the blood flow throughout the blood vessels supporting the heart, i.e. the coronary blood vessels. Those blood vessels may be impaired due to, e.g., thrombotic or atherosclerotic plaques. Accordingly, cardiac interventions shall, preferably, comprise a destruction and/or removal of such plaques and a restoration of the vessel, if necessary. Preferred cardiac interventions in accordance with the present invention are selected from the group consisting of percutaneous coronary angioplasty, percutaneous transluminal coronary balloon angioplasty, laser angioplasty, coronary stent implantation, bypass implantation or intraluminal techniques aiming to restore blood flow, vessel patency, stabilize plaque, and/or reduce intracoronary thrombus load.

In accordance with the present invention, a PCI is to be deemed "successful" if the perfusion, i.e. the blood flow rate, through the coronary blood vessel after PCI, i.e. after the removal of the stenosis or occlusion, is significantly increased compared to the perfusion prior to PCI. Whether an increase in perfusion is statistically significant can be determined by techniques well known in the art and, e.g., described elsewhere in this specification. Preferably, the increase in perfusion observed after PCI allows for restoration of oxygen supply in the ischemic cardiac tissue areas which were previously affected by the coronary heart disease. Thus, subsequent cardiovascular events can be prevented. Preferably, the perfusion after PCI is at least 20%, at least 30%, at least 40%, at least 50%, at least 60%, at least 70%, at least 80%, at least 90% or at least 100% restored.

The term "stable coronary heart diseases" as used herein encompass coronary artery diseases including stenosis, atherosclerosis of the coronary vessels or occlusions, such as thrombo-embolic occlusions within the coronary vessel system. The said coronary heart diseases shall be stable in that no progression or worsening of the symptoms accompanied with the said diseases will be observed for the subject suffering therefrom.

The term "subject" as used herein relates to animals, preferably mammals, and, more preferably, humans. The subject referred to in accordance with the aforementioned method suffers from a stable coronary heart diseases or exhibits the symptoms accompanied therewith, i.e. being at least suspect to suffer from a stable coronary heart disease.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

A "first sample" as referred to in accordance with the present invention is a sample which has been obtained after PCI and, preferably, immediately after PCI. Immediately after PCI, preferably, means within a time frame of 2 to 24 hours, more preferably, within a time frame of 4 to 16 hours, 4 to 12 hours, 5 to 10 hours, or 6 to 8 hours. Most preferably, the sample has been obtained after 6 hours. A "second sample", as referred to herein, is a sample which has been obtained prior to PCI. Such a second sample may be obtained from the subject suffering from a stable coronary heart disease which shall be investigated by the method of the present invention at any time point after the onset of the said stable coronary heart disease. Preferably, it will be obtained at the time point of hospitalization prior to the PCI.

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated gene-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as an approx. 28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

Determining the amount of the peptides or polypeptides referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.
First, binding of a ligand may be measured directly, e.g. by NMR or surface plasmon resonance.
Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method.

Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immune assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the first sample to be analyzed with an amount of the said same peptide or polypeptide in the second sample. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. A computer program may further evaluate the result of the comparison, i.e. automatically provide the desired assessment in a suitable output format. Based on the comparison of the aforementioned amounts, it is possible to assess whether PCI was successful, or not.

Preferably, an amount of GDF-15 in the first sample larger than (i.e. increased or enlarged with respect to) the reference amount (i.e. the amount in the second sample) is indicative for a successful PCI. In contrast, a non-elevated or decreased GDF-15 amount in the first sample versus the second sample will be indicative for an unsuccessful PCI and, thus, may suggest a supplementary therapy as specified elsewhere in this description. An increase in the amount as referred to herein, preferably, is a statistically significant increase. Whether an increase is statistically significant can be determined by techniques well known in the art and referred to elsewhere in this specification. More preferably, it is an increase of at least 20%, at least 30%, at least 40%, at least 50%, at least 60%, at least 70%, at least 80%, at least 90%, at least 100%, at least 250%, at least 500%, at least 750% or at least 1000%.

Advantageously, it has been found in accordance with the present invention that GDF-15 is a suitable marker for assessing the success of a PCI. Accordingly, instead of using expensive and time-consuming monitoring techniques such as angiography which are associated with potentially severe side effects, the method of the present invention allows for a fast, reliable, cost-effective and safe assessment of testing the success of the intervention. Moreover, it is to be understood that a PCI which is not successful may even lead to reduced perfusion in particular of the distal part of the blood vessel, e.g., due to an embolism within the proximal part of the blood vessel where the PCI was carried out. Moreover, PCI may cause thrombosis at the side of PCI or thrombosis in combination embolism whereby the perfusion would also be significantly reduced. The reduced perfusion will cause cardiac necrosis and, as a consequence thereof, further cardiac complications. Thus, the method of the present invention will be applied for assessing whether a supplementary therapy, preferably anti-thrombosis therapy or therapy of embolism, will be necessary after PCI. In this context it is to be understood that a unsuccessful PCI as indicated by the method of the present invention will be an indicator for the necessity of a supplementary therapy as referred to before.

The present invention also, in principle relates to the use of means for determining the amount of GDF-15 and, preferably, means for determining the amount of a natriuretic peptide and/or a cardiac Troponin for the preparation of a diagnostic composition for diagnosing whether a percutaneous cardiac intervention (PCI) in a subject suffering from a stable coronary heart disease was successful.

The definitions and explanations of the terms given above apply mutatis mutandis for the preferred methods, the devices and kits referred to in the following.

In a preferred embodiment of the method of the present invention, said method further comprises:
a) determining the amount of a natriuretic peptide in the said first sample of the said subject; and
b) comparing the amount of the natriuretic peptide determined in step a) to a reference amount of the natriuretic peptide which is determined in the second sample of the said subject suffering from a stable coronary heart disease obtained prior to PCI, whereby it is diagnosed whether PCI was successful.

The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label to the peptide.

More preferably, amount of the natriuretic peptide which is not enlarged with respect to the reference amount is indicative for a successful PCI.

Specifically, it has been found that a successful PCI is accompanied with persisting, i.e. not elevated, amounts of the natriuretic peptide and, in particular, NT-proBNP, in the first and the second sample. In contrast, if the PCI was unsuccessful, the amount of the natriuretic peptide and, preferably, NT-proBNP to be determined in the first sample will be increased with respect to the second sample (i.e. the reference amount).

In a further preferred embodiment of the method of the present invention, said method further comprises the steps of
a) determining the amount of a cardiac Troponin in the said first sample of the said subject; and
b) comparing the amount of the cardiac Troponin determined in step a) to a reference amount of the cardiac Troponin which is determined in the second sample of the said subject suffering from a stable coronary heart disease obtained prior to PCI, whereby it is diagnosed whether the PCI was successful.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Tropoinin T or Troponin I. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Troponin. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Such fragments may be, e.g., degradation products of the Troponins. Further included are variants which differ due to posttranslational modifications such as phosphorylation or myristylation.

More preferably, an amount for the cardiac Troponin which is not enlarged with respect to the reference amount is indicative for a successful PCI.

Specifically, it has been found that a successful PCI is accompanied with persisting, i.e. not elevated, amounts of the cardiac Troponin and, in particular, Troponin T, in the first and the second sample. In contrast, if the PCI was unsuccessful, the amount of the cardiac Troponin and, preferably, Troponin T to be determined in the first sample will be increased with respect to the second sample (i.e. the reference amount).

The present invention also relates to a device adopted for diagnosing whether a percutaneous cardiac intervention (PCI) in a subject suffering from a stable coronary heart disease was successful, said device comprising:
a) means for determining the amount of GDF-15, preferably in combination with a natriuretic peptide and/or a cardiac Troponin, in a first sample of the said subject which has been obtained after PCI and a second sample which has been obtained prior to PCI; and
b) means for comparing the amount determined by the means of a) for the said first and second sample, thereby allowing to diagnose whether the PCI was successful.

The term "device" as used herein relates to a system of means comprising at least the aforementioned means operatively linked to each other as to allow the prediction. Preferred means for determining the amount of a peptide or polypeptide as well as means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the peptides or polypeptides in an applied sample and a computer unit for processing the resulting data for the evaluation. The computer unit, preferably, comprises a database including the stored reference amounts or values thereof recited elsewhere in this specification as well as a computer-implemented algorithm for carrying out a comparison of the determined amounts for the polypeptides with the stored reference amounts of the database. Computer-implemented as used herein refers to a computer-readable program code tangibly included into the computer unit. Alternatively, where means such as test stripes are used for determining the amount of the peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the peptides, Plasmon surface resonace devices, NMR spectrometers, mass- spectrometers etc.) and/or evaluation units/devices referred to above in accordance with the method of the invention.

Finally, the present invention pertains to a kit adopted for carrying out the methods of the present invention, said kit comprising instructions for carrying out the said method and
a) means for determining the amount of GDF-15, preferably in combination with a natriuretic peptide and/or a cardiac Troponin, in a first sample of the said subject which has been obtained after PCI and a second sample which has been obtained prior to PCI; and
b) means for comparing the amount determined by the means of a) for the said first and second sample, thereby allowing to diagnose whether the PCI was successful.

The term "kit" as used herein refers to a collection of the aforementioned means, preferably, provided separately or within a single container. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

**Example**: Analysis of time-dependent changes of GDF-15, NT-proBNP and Troponin T in patients which have been subjected to percutaneous intervention therapy

In a study, 37 patients suffering from coronary heart disease with an indication for stent implantation have been investigated for their GDF-15, NT-proBNP and sensitive Troponine T levels prior and after the implantation. GDF-15 was determined in blood samples as described by Kempf 2007, Clinical Chemistry 53(2):284-91. NT-proBNP and sensitive Troponine T was determined by the Elecsys^{™} Assay for NT-proBNP and the Elecsys^{™} Assay for sensitive Troponine T (both of Roche Diagnostics, Germany).

5 patients showed no GDF-15 increase while 32 patients showed an increase in the detectable amount of GDF-15 within the first 6 hours after implantation. Patients exhibiting increased GD-15 levels after 6 hours showed no or no significant increase of the NT-proBNP or sensitive Troponine T after 6 hours and 24 hours, respectively. In contrast, patients lacking a GDF-15 increase showed a dramatic increase of both, sensitive Troponine T and NT-proBNP after 6 and 24 hours, respectively. These patients showed no or only an insufficient reperfusion after stent implantation. Accordingly, the implantation measure was not successful. And further supplementary therapies were necessary.

The results are summarized in the following tables:

**Table 1: Patients (n=32) which have been successfully treated by PCI**

| Hours after PCI | 0 | 6 | 24 | 0 | 6 | 24 | 0 | 6 | 24 |
|---|---|---|---|---|---|---|---|---|---|
| biomarker | GDF-15 | GDF-15 | GDF-15 | NTproBNP | NTproBNP | NTproBNP | hsTnT | hsTnT | hsTnT |
| Median | 584 | 851 | 756 | 194 | 239 | 186 | 1 | 17 | 20 |
| 75th perc. | 695 | 1155 | 1016 | 761 | 719 | 650 | 6 | 59 | 91 |
| 95th perc. | 1087 | 1674 | 1958 | 2640 | 2502 | 2417 | 149 | 1519 | 830 |
| 5th perc. | 369 | 517 | 501 | 42 | 30 | 32 | 1 | 1 | 1 |
| 25th perc. | 488 | 703 | 598 | 70 | 85 | 84 | 1 | 1 | 1 |

**Table 2: Patients (n=5) which have not been successfully treated by PCI**

| Hours after PCI | 0 | 6 | 24 | 0 | 6 | 24 | 0 | 6 | 24 |
|---|---|---|---|---|---|---|---|---|---|
| biomarker | GDF-15 | GDF-15 | GDF-15 | NTproBNP | NTproBNP | NTproBNP | hsTnT | hsTnT | hsTnT |
| Median | 548 | 608 | 551 | 155 | 333 | 386 | 1 | 25 | 170 |
| 75th perc. | 553 | 914 | 669 | 286 | 482 | 399 | 348 | 2830 | 2968 |
| 95th perc. | 819 | 1006 | 943 | 385 | 553 | 513 | 2612 | 5349 | 4262 |
| 5th perc. | 507 | 516 | 422 | 145 | 215 | 190 | 1 | 4 | 51 |
| 25th perc. | 540 | 569 | 547 | 153 | 290 | 258 | 1 | 8 | 100 |

Concentrations of GDF-15, sensitive Troponin T and NT-proBNP are indicated in pg/ml in the above tables.

## Claims

1. A method for diagnosing whether a percutaneous cardiac intervention (PCI) in a subject suffering from a stable coronary heart disease was successful, said method comprising:
a) determining the amount of GDF-15 in a first sample of the said subject which has been obtained after PCI; and
b) comparing the amount of GDF-15 determined in step a) with a reference amount of GDF-15 which is determined in a second sample of the said subject suffering from a stable coronary heart disease obtained prior to PCI, whereby it is diagnosed whether PCI was successful.

2. The method of claim 1, wherein an amount of GDF-15 larger than the reference amount is indicative for a successful PCI.

3. The method of claim 1 or 2, wherein said method further comprises:
a) determining the amount of a natriuretic peptide in the said first sample of the said subject; and
b) comparing the amount of the natriuretic peptide determined in step a) to a reference amount of the natriuretic peptide which is determined in the second sample of the said subject suffering from a stable coronary heart disease obtained prior to PCI, whereby it is diagnosed whether PCI was successful.

4. The method of claim 3, wherein an amount of the natriuretic peptide which is not enlarged with respect to the reference amount is indicative for a successful PCI.

5. The method of any one of claims 1 to 4, wherein said method further comprises the steps of
a) determining the amount of a cardiac Troponin in the said first sample of the said subject; and
b) comparing the amount of the cardiac Troponin determined in step a) to a reference amount of the cardiac Troponin which is determined in the second sample of the said subject suffering from a stable coronary heart disease obtained prior to PCI, whereby it is diagnosed whether the PCI was successful.

6. The method of claim 5, wherein an amount for the cardiac Troponin which is not enlarged with respect to the reference amount is indicative for a successful PCI.

7. The method of any one of claims 1 to 6, wherein the said first sample is to be obtained within 2 to 24 hours after PCI.

8. The method of claim 7, wherein the first sample is to be obtained 6 hours after PCI.

9. The method of any one of claims 3 to 8, wherein the said natriuretic peptide is a BNP-type peptide.

10. The method of claim 9, wherein said BNP-type peptide is NT-proBNP.

11. The method of any one of claims 5 to 10, wherein the said cardiac Troponin is Troponin T.

12. The method of any one of claims 1 to 11, wherein the said subject is a human.

13. A device adopted for diagnosing whether a percutaneous cardiac intervention (PCI) in a subject suffering from a stable coronary heart disease was successful, said device comprising:
a) means for determining the amount of GDF-15, preferably in combination with a natriuretic peptide and/or a cardiac Troponin, in a first sample of the said subject which has been obtained after PCI and a second sample which has been obtained prior to PCI; and
b) means for comparing the amount determined by the means of a) for the said first and second sample, thereby allowing to diagnose whether the PCI was successful.

14. Use of means for determining the amount of GDF-15 and, preferably, means for determining the amount of a natriuretic peptide and/or a cardiac Troponin for the preparation of a diagnostic composition for diagnosing whether a percutaneous cardiac intervention (PCI) in a subject suffering from a stable coronary heart disease was successful.

15. A kit adopted for carrying out the methods of any one of claims 1 to 12, said kit comprising instructions for carrying out the said method and
a) means for determining the amount of GDF-15, preferably in combination with a natriuretic peptide and/or a cardiac Troponin, in a first sample of the said subject which has been obtained after PCI and a second sample which has been obtained prior to PCI; and
b) means for comparing the amount determined by the means of a) for the said first and second sample, thereby allowing to diagnose whether the PCI was successful.
